# EUROPÄISCHE PATENTSCHRIFT

(11) **EP 3 475 032 B1**
(45) Veröffentlichungstag und Bekanntmachung des Hinweises auf die Patenterteilung: **22.12.2021**
(21) Anmeldenummer: 17733434.9
(22) Anmeldetag: 26.06.2017
(51) Int. Cl.: B25J 9/00, A61B 6/04, B25J 9/04

(54) **ROBOTER UND ROBOTERANORDNUNG ZUR PATIENTENPOSITIONIERUNG**
ROBOT AND ROBOT ASSEMBLY FOR PATIENT POSITIONING
ROBOT ET ENSEMBLE ROBOTIQUE POUR LE POSITIONNEMENT DE PATIENTS

(30) Priorität: 27.06.2016 DE 102016211538
(43) Veröffentlichungstag der Anmeldung: 01.05.2019
(73) Patentinhaber: LEONI Kabel GmbH, 91154 Roth (DE)
(72) Erfinder: PINAULT, Samuel, 78460 Chevreuse (FR)
(74) Vertreter: Frenkel, Matthias Alexander
(86) Internationale Anmeldenummer: PCT/EP2017/065688
(87) Internationale Veröffentlichungsnummer: WO 2018/001951

(56) Entgegenhaltungen:
- EP-A1- 2 745 997
- DE-A1-102007 046 080
- DE-A1-102009 035 637
- US-A- 5 155 423
- US-A1- 2011 066 278

## Beschreibung

Die Offenbarung betrifft einen Roboter und eine Roboteranordnung sowie eine Roboterarbeitszelle zur Patientenpositionierung.

Bei einer bildgebenden medizinischen Untersuchung, wie beispielsweise Röntgen, Computertomografie oder Kernspinresonanz, sowie bei einer medizinischen Strahlentherapie müssen Patienten und die entsprechenden medizinischen Vorrichtungen in gewünschter Weise relativ zueinander positioniert und/oder zueinander bewegt werden. Beispielsweise muss bei der Strahlentherapie sichergestellt werden, dass nur erkranktes Gewebe bestrahlt wird nicht aber gesundes. Gerade für ältere oder beeinträchtigte Patienten ist zur genauen Positionierung ein hoher Aufwand erforderlich und eine maximale Genauigkeit / Positionierung mit minimaler Abweichung lässt sich nicht erzielen, so dass hierdurch oftmals ein größerer Bereich bestrahlt werden muss und hierdurch beispielsweise auch gesundes Gewebe in Mitleidenschaft gerät.

Existierende kinematische Systeme zur Patientenpositionierung, beispielsweise in Form einer linear verschieblichen Patientenliege, stellen hingegen nur beschränkte Bewegungsmöglichkeiten des Patienten bereit.

Das Dokument EP 2 745 997 A1 zeigt deckenmontierte Roboter innerhalb einer Vakuumkammer, die an einer rampenförmigen Struktur angebracht sind.

Aus dem Dokument DE 10 2007 046080 A1 ist ein Strahlentherapieraum bekannt, bei dem eine Patientenliege beweglich mittels eines Industrieroboters gelagert ist. Letzterer ist über eine rampenförmige Struktur mit einem Bodenbereich verbunden.

Aus dem Dokument US 2011/066278 A1 ist ein Patientenpositioniersystem bekannt, bei dem ein Roboter eine Patientenliege trägt und an einer Linearachse montiert ist.

Das Dokument DE 10 2009 035 637 A1 offenbart einen Roboter mit einem rampenförmigen Sockelelement, der zum Be- oder Entladen einer Werkzeugmaschine vorgesehen ist.

Schließlich zeigt das Dokument US 5 155 423 A ebenfalls einen an einem rampenförmigen Sockel angebrachten Roboter.

Es besteht somit ein Bedarf, ein System bereitzustellen, das eine verbesserte Positionierung und/oder Bewegung eines Patienten relativ zu medizinischen Vorrichtungen ermöglicht.

Erfindungsgemäß wird hierfür ein Roboter, beispielsweise zur Patientenpositionierung, gemäß Anspruch 1 bereitgestellt.

Der Roboter umfasst einen Roboterarm mit einer Mehrzahl von Robotergliedern, die über Achseinheiten miteinander verbunden sind. Die Achseinheiten definieren jeweils wenigstens eine Bewegungsachse des Roboterarms. Der Roboterarm umfasst einen ersten Endbereich, der ein Anordnen an einem eine im Wesentlichen horizontale Ebene umfassenden Umgebungsbereich des Roboters ermöglicht, und einen zweiten Endbereich, an dem ein Endeffektor anordenbar ist. Eine dem ersten Endbereich nachgeordnete erste Achseinheit definiert eine erste Rotationsachse des Roboterarms. Der Roboterarm ist mittels des ersten Endbereichs derart an dem Umgebungsbereich anordenbar, dass die erste Rotationsachse in einem Winkel schräg zu dem Umgebungsbereich verläuft. Anders ausgedrückt, kann der Roboterarm mittels des ersten Endbereichs derart an dem Umgebungsbereich anordenbar sein / angeordnet werden, dass die erste Rotationsachse in einem Winkel geneigt zu dem Umgebungsbereich verläuft. Unter der Neigung zu dem Umgebungsbereich kann beispielsweise jede von 0° und 90° abweichende Neigung verstanden werden.

Mit anderen Worten haben die Erfinder einen Roboter mit einer neuartigen kinematischen Struktur entwickelt, die es ermöglicht, Patienten besonders präzise und aufwandsarm relativ zu medizinischen Vorrichtungen der eingangs genannten Art zu positionieren. Wie nachfolgend erläutert, zeichnet sich diese kinematische Struktur insbesondere durch eine hohe Robustheit, Kompaktheit und Zuverlässigkeit aus, da beispielsweise Kollisionen mit der Umgebung besser vermieden werden können. Ebenso ermöglicht sie ein besonders bodennahes Anordnen des Endeffektors und somit einen entsprechend vergrößerten Arbeitsbereich.

Bei den Achseinheiten kann es sich in bekannter Weise um motorisch angetriebene Gelenkeinheiten handeln, die Linear -und/oder Rotationsachsen definieren. Diese können jeweils Bewegungsachsen bilden, um beziehungsweise entlang derer sich die mit den Achseinheiten gekoppelten Roboterglieder bewegen können. Zumindest die erste Rotationsachse kann allgemein durch ein angetriebenes Rotationsgelenk gebildet werden und hierzu einen Motor, beispielsweise einen Servomotor, umfassen. In einer spezifischen Ausführungsform besitzt der Roboterarm ausschließlich Rotationsachsen, und kann, wie nachfolgend erläutert, zusätzlich insgesamt auf einer Linearachse angeordnet sein, die den Umgebungsbereich bildet.

Die Roboterglieder können in bekannter Weise starre Abschnitte bilden, die mit den entsprechenden Achseinheiten gekoppelt sind und einzelne kinematische Glieder des Roboterarms bilden. Beispielsweise kann der Roboterarm allgemein eine offene kinematische Kette aufweisen, wobei die Roboterglieder die einzelnen kinematischen Glieder dieser Kette bilden. Dabei kann sich die kinematische Kette von dem beispielsweise starr an den Umgebungsbereich gekoppelten ersten Endbereich in den Raum erstrecken und mittels seiner Bewegungsachsen den zweiten Endbereich des Roboterarms sowie den damit gekoppelten Endeffektor in gewünschter Weise im Raum positionieren. Allgemein kann ferner vorgesehen sein, ausgehend von dem ersten Endbereich die Achseinheiten sowie die davon festgelegten Bewegungsachsen aufsteigend zu nummerieren. Im Fall einer offenen kinematischen Kette kann eine nächsthöhere Achseinheit der vorangehenden Achseinheit jeweils unmittelbar nachgeordnet sein. Beispielsweise folgt die dritte Rotationsachse auf die zweite Rotationsachse, die wiederum der ersten Rotationsachse nachgeordnet ist, wobei die jeweiligen Rotationsachsen über einzelne Roboterglieder miteinander verbunden sind.

Die Roboterglieder und/oder die Achseinheiten können ferner hohle Bereiche umfassen, um darin Kabel, Leitungen oder dergleichen zu führen. Dies ermöglicht, dass keine oder nur wenige Kabel welche die Störkontur des Roboters vergrößern oder sich in der Umgebung verhaken könnten, außen entlang des Roboterarms geführt werden müssen.

Bei dem Endeffektor handelt es sich zum Beispiel um eine Handhabungseinheit und/oder insbesondere um eine Patientenliege, die eine Liegefläche für einen Patienten aufweisen kann. Dabei kann ein Anschlussbereich der Patientenliege zu dem zweiten Endbereich des Roboterarms außerhalb eines zentralen Bereichs der Liegefläche angeordnet sein, und insbesondere außerhalb einer geometrischen Mitte der Liegefläche. Mit anderen Worten kann der Roboterarm mit der Patientenliege in einem exzentrischen Bereich von dessen Liegefläche gekoppelt sein. Bildlich gesprochen kann die Patientenliege somit über den Roboterarm frei hervorstehen beziehungsweise von diesem auskragen. Ferner kann der Roboterarm an einer Unterseite der Patientenliege mit dieser gekoppelt sein und die Patientenliege allgemein von unten anheben beziehungsweise stützen.

Für das Anordnen an dem Umgebungsbereich kann der erste Endbereich ferner in bekannter Weise einen Kopplungsbereich und/oder geeignete Kopplungselemente umfassen, beispielsweise in Bohrungen in dem ersten Endbereich einsetzbare Schraubbolzen. Ebenso kann der Kopplungsbereich eine Kopplung mit einer nachfolgend erläuterten Linearachseinheit ermöglichen, um den Roboterarm zusätzlich im Raum verfahren zu können. Das Anordnen kann somit ein mechanisches Koppeln mit der Umgebung einschließen und insbesondere ein Befestigen daran.

Durch die Wahl des vorstehend genannten Winkels für die erste Rotationsachse ist diese allgemein geneigt beziehungsweise zwischen einer horizontalen und einer vertikalen Raumebene anordenbar. Der Umgebungsbereich kann ferner eine beispielsweise planare Ebene definieren, zu der die Rotationsachse entsprechende geneigt ist. Mit anderen Worten kann sich der Roboterarm sozusagen schief oder schräg relativ zu dem Umgebungsbereich erstrecken.

Hierdurch kann insgesamt eine kompakte Gestalt des Roboters erzielt werden und, insbesondere bei einer Verwendung zur Patientenpositionierung, eine besonders geeignete Bewegbarkeit sowie ein bevorzugtes Kraft-/ Drehmomentübertragungsverhalten. Beispielsweise haben die Erfinder erkannt, dass die schräg verlaufende erste Rotationsachse sogenannte Backlash-Fehler reduziert und ferner bei kompakter Gestaltung besonders zuverlässig mit exzentrischen beziehungsweise Auslegerähnlichen Lasteinleitungsbereichen umgehen kann. Somit können auch die Antriebseinheiten der einzelnen Achseinheiten (zum Beispiel in Form von Motoren, wie Servomotoren) kompakter sowie weniger leistungsstark ausgebildet werden oder aber bei gleicher oder kompakterer Gestalt höhere Lasten bewegen.

Insbesondere haben die Erfinder erkannt, dass die vorliegende Kinematik vorteilhaft für das Stützen und Anheben von Lasten durch eine unterseitige Krafteinleitung ist (also bildlich gesprochen für ein Stützen oder Anheben der Lasten von unten). Dieser Lastfall geht insbesondere mit auf den Roboterarm einwirkenden hohen Druck- sowie Biegekräften einher.

In diesem Zusammenhang kann ferner vorgesehen sein, dass die erste Rotationsachse in einem Winkel zwischen ca. 10° und ca. 80°, zum Beispiel in einem Winkel von ca. 20° bis ca. 70° und beispielsweise ca. 45°, zu dem Umgebungsbereich verläuft. Ebenso kann vorgesehen sein, dass die erste Rotationsachse in einem Winkel von ca. 30 bis ca. 60° oder ca. 40° bis ca. 50° zu dem Umgebungsbereich verläuft.

Gemäß einer Weiterbildung ist vorgesehen, dass der erste Endbereich eine Anschlussebene umfasst, die einen selben Winkel mit der ersten Rotationsachse einschließt wie der Umgebungsbereich. Beispielsweise kann die Anschlussebene sich dabei parallel zu dem Umgebungsbereich erstrecken. Mit anderen Worten kann demnach vorgesehen sein, dass die erste Achseinheit oder zumindest dessen erste Rotationsachse auch innerhalb der Struktur des Roboters geneigt ist, insbesondere bezogen auf die Anschlussebene des ersten Endbereichs. Dabei kann die Anschlussebene den vorstehend geschilderten Kopplungsbereich des ersten Endbereichs umfassen oder bilden. Insbesondere kann die Anschlussebene ein plattenförmiges Bauteil umfassen, das ferner an dessen Oberseite mit der ersten Achseinheit verbunden sein kann und mit dessen Unterseite dem Umgebungsbereich zugewandt ist. Die Unter- und die Oberseite erstrecken sich dabei beispielsweise im Wesentlichen parallel zueinander.

Erfindungsgemäß ist vorgesehen, dass der Umgebungsbereich eine im Wesentlichen horizontale Raumebene und beispielsweise eine Bodenebene umfasst. Genauer gesagt ist der Roboter allgemein dazu ausgebildet, den Roboterarm mittels des ersten Endbereichs an einer horizontalen Ebene und insbesondere an einer Bodenebene zu befestigen. Ebenso kann die horizontale Raumebene durch eine nachfolgend erläuterte Linearachseinheit gebildet werden, mittels derer der Roboter im Raum verfahrbar ist.

Gemäß einem weiteren Aspekt ist die Anschlussebene über wenigstens zwei Kopplungselemente mit dem Umgebungsbereich gekoppelt und ein Verbindungsbereich der ersten Achseinheit mit einem ersten Roboterglied ist im Wesentlichen zwischen den Kopplungselementen angeordnet. Wie bereits erwähnt, kann es sich bei den Kopplungselementen um Befestigungselemente wie Schraubbolzen und/oder Elemente zur Kopplung mit einer weiteren Linearachseinheit handeln, an der der Roboter angeordnet ist, wie beispielsweise Lager- oder Führungselemente und/oder Antriebseinheiten (beispielsweise Linearmotoren) oder damit koppelbare Einheiten.

Durch das Anordnen des Verbindungsbereiches zwischen den Kopplungselementen kann eine Krafteinleitung in den Umgebungsbereich und somit das Abstützen des Roboters an der Umgebung verbessert werden. Insbesondere können somit die Stabilität beeinträchtigende Hebelarme reduziert werden. Der Begriff "zwischen" kann sich dabei auf ein Anordnen in einem beliebigen Zwischenraum zwischen den beiden Kopplungselementen beziehen. Insbesondere können der Verbindungsbereich und die Kopplungselemente auf verschiedenen Höhenniveaus liegen / verschiedene Höhenniveaus aufweisen. Ebenso kann darunter verstanden werden, dass bei einem Projizieren in eine gemeinsame Ebene und Verbinden der Kopplungselemente mittels einer gedachten Linie der Verbindungsbereich an einer beliebigen Position entlang dieser Linie und somit zwischen den Kopplungselementen liegt (nicht aber zwangsläufig unmittelbar auf dieser Linie).

Man beachte, dass durch die Schrägstellung der ersten Rotationsachse die Stabilität beeinträchtigende Hebelarme auch deshalb reduziert werden können, da die Verbindungstelle zu dem ersten Roboterglied in einem reduzierten vertikalen Abstand zu dem Umgebungsbereich anordenbar ist. Dies gilt insbesondere im Vergleich zu bekannten Roboterkinematiken mit einer aufrechten vertikalen ersten Rotationsachse.

In diesem Zusammenhang kann ferner vorgesehen sein, dass die Anschlussebene wenigstens vier Kopplungselemente umfasst, die in verschiedenen Eckbereichen der Anschlussebene angeordnet sind und einen im Wesentlichen rechteckförmigen Bereich aufspannen. Hierbei kann die Anschlussebene insbesondere ein plattenförmiges Bauteil mit entsprechenden Eckbereichen umfassen. In diesem Fall kann der Verbindungsbereich der ersten Achseinheit im Wesentlichen im Bereich eines Diagonalenschnittpunktes des rechteckförmigen Bereiches angeordnet sein. Ebenso kann allgemein vorgesehen sein, dass der Verbindungsbereich im Wesentlichen mittig zwischen wenigstens zwei der Kopplungselemente angeordnet ist.

Eine Weiterbildung sieht vor, dass der Roboterarm ferner eine der ersten Achseinheit nachgeordnete zweite Achseinheit umfasst, die eine zweite Rotationsachse des Roboterarms definiert. Wie erläutert, kann es sich hierbei insbesondere um eine innerhalb einer kinematischen Kette des Roboterarms unmittelbar nachgeordnete Achseinheit handeln und die Achseinheiten sind beispielsweise über ein gemeinsames Roboterglied miteinander gekoppelt.

In diesem Zusammenhang kann der Roboterarm dazu ausgebildet sein, die zweite Achseinheit nahe des Umgebungsbereiches zu positionieren, beispielsweise derart, dass sie dem Umgebungsbereich unmittelbar gegenüberliegt. Hierzu kann ein Rotationsbereich der ersten Achseinheit entsprechend gewählt und/oder ein die erste und zweite Achseinheit verbindendes Roboterglied entsprechend gestaltet sein. Zum Beispiel ist dieses Roboterglied jedoch im Wesentlichen geradlinig (und/oder zylindrisch). Demnach kann vorgesehen sein, dass der Roboterarm derart über seine Achseinheiten ausrichtbar ist, dass die zweite Achseinheit insbesondere nahe eines Bodenbereiches anordenbar ist, mit dem der Roboter gekoppelt ist, sowie allgemein nahe des ersten Endbereiches des Roboters. Hierbei können allgemein Abstände zwischen ca. 2 cm und ca. 50 cm und beispielsweise ca. 5 cm und ca. 20 cm oder ca. 2 cm und 10 cm zu dem entsprechenden Umgebungsbereich vorgesehen sein.

In diesem Zusammenhang kann ferner vorgesehen sein, dass die erste und zweite Rotationsachse des Roboterarms um ca. 90° relativ zueinander verdreht sind und allgemein, dass diese durch eine Gerade verbindbar sind, die im Wesentlichen senkrecht auf beiden Rotationsachsen steht. Die verbindende Gerade kann sich dabei entlang eines die entsprechenden Achseinheiten verbindenden Robotergliedes erstrecken. Mit anderen Worten können die erste und zweite Rotationsachse (zumindest bei einem Projizieren in eine gemeinsame Ebene) im Wesentlichen senkrecht relativ zueinander verlaufen. Im Ergebnis kann somit auch die zweite Rotationsachse zu dem Umgebungsbereich geneigt sein oder mit einer entsprechenden Ausrichtung relativ zu dem Umgebungsbereich anordenbar sein.

Durch die kinematische Abfolge einer schräg verlaufenden ersten Rotationsachse sowie einer hierzu im Wesentlichen senkrechten zweiten Rotationsachse wird auf kompaktem Raum eine besonders große Beweglichkeit erzielt, insbesondere bei Anordnen des Roboters an einem horizontalen Umgebungsbereich und insbesondere für die Patientenpositionierung relativ zu medizinischen Vorrichtungen. Wie nachfolgend erläutert, kann der Roboter demnach auch besonders bodennah ausgebildet und betreibbar sein, was die Kollisionsgefahr mit benachbarten Vorrichtungen mindert. Im Fall einer an dem Roboterarm angeordneten Patientenliege ermöglicht dies auch einen einfachen und komfortablen Zugang zu der Patientenliege, da diese ebenfalls in einem geringen Abstand zum Boden anordenbar ist.

Ferner kann der Roboter eine der ersten und zweiten Achseinheit nachgeordnete dritte Achseinheit umfassen, die eine entsprechende dritte Rotationsachse des Roboterarms definiert, und, optional, ferner der dritten Achseinheit nachgeordnete vierte und fünfte Achseinheiten, die entsprechende vierte und fünfte Rotationsachsen des Roboterarms definieren. Wie im vorstehenden Fall können die jeweiligen Achseinheiten innerhalb einer kinematischen Kette unmittelbar aufeinanderfolgen und/oder mittels gemeinsamer Roboterglieder miteinander gekoppelt sein. Insgesamt kann vorgesehen sein, dass der Roboterarm demnach nicht mehr als fünf Rotationsachsen umfasst, wobei diese zum Beispiel die einzigen Bewegungsachsen innerhalb des Roboterarms bilden. Die Erfinder haben erkannt, dass diese Anzahl von Roboterachsen insbesondere im Zusammenspiel mit der vorstehend geschilderten schrägen Grundkinematik eine zuverlässige und ausreichende Bewegbarkeit für die Patientenpositionierung ermöglicht, wobei gleichzeitig eine kompakte Gestalt des Roboters beibehalten werden kann.

Die erste und die zweite Achseinheit können dabei über ein Turretsegment (abgeleitet von dem Englischen "turret segment") gekoppelt sein.

Die dritte, vierte und fünfte Achseinheit können eine allgemein bekannte Roboterhandgelenkseinheit bilden. Zum Beispiel sind die Rotationsachsen dieser (dritten bis fünften) Achseinheiten ferner zumindest zu der unmittelbar vorangehenden Rotationsachse jeweils orthogonal ausgerichtet. Ebenso können sämtliche Rotationsachsen dieser Achseinheiten jeweils orthogonal zueinander verlaufen oder derart ausrichtbar sein, zumindest während eines Normalbetriebs des Roboters (Anheben und Stützen einer Patientenliege von unten).

In diesem Zusammenhang kann ferner vorgesehen sein, dass die dritte Rotationsachse im Wesentlichen senkrecht zu der zweiten Rotationsachse verläuft.

Gemäß einem weiteren Aspekt sind die zweite und dritte Achseinheit zwischen ca. 30 cm und ca. 2 m voneinander beabstandet und beispielsweise zwischen ca. 60 cm und ca. 1,50 m. Ebenso kann ein Abstand von ca. 60 cm bis ca. 1,20 m vorgesehen sein. Gemäß dieser Variante können die dritte Achseinheit und gegebenenfalls weitere damit verbundene Achseinheiten, die gemeinsam beispielsweise eine Handgelenkseinheit des Roboterarms bilden, somit in einem gewissen Mindestabstand zu der ersten und zweiten Achseinheit angeordnet sein. Der Abstand kann dabei von einem die zweite und dritte Achseinheit verbindenden Roboterglied definiert und/oder entlang diesem gemessen werden.

Dies führt im Ergebnis dazu, dass die erste und zweite Achseinheit beabstandet von dem zweiten Endbereich des Roboterarms angeordnet werden können, was vorteilhaft bezüglich einer Kollisionsvermeidung sein kann. Zum Beispiel kann hierdurch ermöglicht werden, dass der zweite Endbereich des Roboters beabstandet von den vergleichsweise groß bauenden ersten und zweiten (Basis-) Achseinheiten anordenbar ist und somit in dessen Nähe vergleichsweise viel freier Raum zur Verfügung steht. Mit anderen Worten kann der Roboter nahe des zweiten Endbereichs eine vergleichsweise geringe Baugröße und somit reduzierte Störkontur aufweisen. Folglich kann dieser Endbereich auch in vergleichsweise engen Arbeitsbereichen positioniert werden, beispielsweise in der Nähe oder innerhalb von medizinischen Vorrichtungen.

Die Offenbarung betrifft ferner eine Roboteranordnung, umfassend einen Roboter nach einem der vorangehenden Aspekte, wobei der Umgebungsbereich eine Linearachseinheit umfasst (und/oder durch diese gebildet wird), an der der Roboterarm über dessen ersten Endbereich anordenbar ist. Hierdurch kann der Gesamtarbeitsraum des Roboters vergrößert werden und insbesondere eine allgemein zylindrische Form annehmen.

Die Linearachseinheit kann in bekannter Weise mit dem Roboter gekoppelt werden, und diesen linear im Raum verschieben. Wie bereits angedeutet, kann dies mittels einer Anschlussebene und/oder eines Kopplungsbereiches des ersten Endbereichs des Roboterarms erfolgen. Ferner können hierzu Kopplungselemente, wie beispielsweise Führungs- oder Lagerelemente oder aber auch Antriebseinheiten z.B. in Form von Linearmotoren oder damit koppelbare Einheiten verwendet werden, sodass der Roboter beispielsweise entlang einer Führungsschiene der Linearachseinheit verfahrbar ist.

In diesem Zusammenhang kann die Linearachseinheit eine lineare Bewegungsachse definieren, die einen selben Winkel mit der ersten Rotationsachse des Roboters einschließt wie der Umgebungsbereich. Mit anderen Worten kann die lineare Bewegungsachse, entlang derer der Roboter verschieblich ist, sich parallel zu einem beispielsweise horizontalen Umgebungsbereich erstrecken und/oder zu einer Anschlussebene des ersten Endbereichs des Roboterarms. Allgemein kann die erste Rotationsachse des Roboterarms somit ebenfalls schräg zu der linearen Bewegungsachse verlaufen. Man beachte, dass die lineare Bewegungsachse in bekannter Weise eine Translation des Roboters innerhalb einer Ebene ermöglichen, hierbei jedoch auch einen kurvenförmigen oder gekrümmten Bewegungspfad definieren kann.

Ebenso kann vorgesehen sein, dass die Linearachseinheit an einem Bodenbereich anordenbar ist. Auch in diesem Fall kann somit gewährleistet werden, dass der Roboter besonders bodennah operieren kann und somit eine reduzierte Störkontur sowie durch die schräg verlaufende erste Rotationsachse eine bevorzugte Bewegbarkeit aufweist.

Gemäß einem weiteren Aspekt kann eine an sich bekannte Kalibration der kinematischen und/oder elastischen Eigenschaften des Roboters (und insbesondere von dessen Roboterarm) an mehreren Positionen der Linearachse und beispielsweise in gleichmäßigen Intervallen entlang der Linearachse durchgeführt werden. Über Interpolationen oder dergleichen können somit für jede Position entlang der Linearachse entsprechende Kalibrierungsdaten gewonnen und hinterlegt werden, auf die bei der Steuerung des Roboters zurückgegriffen werden kann. Somit kann eine hohe Positionierungsgenauigkeit entlang der gesamten Linearachse gewährleistet werden, da lokale Wechselwirkungen zwischen dem Roboter und der Linearachse berücksichtigt werden können.

Die Linearachseinheit kann ferner modular aufgebaut sein, beispielsweise in Form von einzelnen Modulen mit ca. 1 m Länge, um die Erstreckung der Linearachse flexibel anzupassen.

Schließlich betrifft die Offenbarung auch eine Roboterarbeitszelle, umfassend einen Bodenbereich und eine Roboteranordnung nach einem der vorangehenden Aspekte, wobei die Linearachseinheit an dem Bodenbereich angeordnet ist. In diesem Zusammenhang kann ferner vorgesehen sein, dass die Roboterarbeitszelle auch eine bildgebende medizinische Vorrichtung und/oder eine Vorrichtung zur medizinischen Strahlentherapie umfasst, wobei die Roboteranordnung dazu ausgebildet ist, einen Patienten in einem Arbeitsbereich der medizinischen Vorrichtung zu positionieren.

Hierzu kann vorgesehen sein, die Roboteranordnung und insbesondere dessen Linearachseinheit derart innerhalb der Roboterarbeitszelle sowie relativ zu den etwaigen medizinischen Vorrichtungen anzuordnen, dass sich die Arbeitsbereiche der Roboteranordnung und der medizinischen Vorrichtung in gewünschter Weise überschneiden. Insbesondere kann hierbei vorgesehen sein, dass die Roboteranordnung in der vorstehend beschriebenen Weise mit einer Patientenliege gekoppelt und dazu ausgebildet ist, die Patientenliege in gewünschter Weise relativ zu den Vorrichtungen zu positionieren.

Ein Beispiel sieht vor, dass eine bildgebende medizinische Vorrichtung derart relativ zu der Linearachseinheit angeordnet ist, dass ein mittels des Roboters ausgerichteter Patient über ein Antreiben der Linearachse in den Arbeitsbereich der medizinischen Vorrichtung hineingeschoben werden kann. Dies betrifft beispielsweise bekannte Computertomografen, in die der Patient zumindest teilweise hineingeschoben werden muss. Hierfür kann die medizinische Vorrichtung an oder benachbart zu einem (axialen) Endbereich der Linearachse angeordnet sein.

Gemäß einer weiteren Variante kann vorgesehen sein, dass die Roboterarbeitszelle mehrere derartige medizinischen Vorrichtungen umfasst und die Roboteranordnung dazu ausgebildet ist, insbesondere mittels einer geeigneten Erstreckung der Linearachseinheit den Patienten in gewünschter Weise relativ zu sämtlichen medizinischen Vorrichtungen zu positionieren. Dies ermöglicht eine erhöhte Flexibilität innerhalb eines Behandlungsraumes, da ein Patient aufwandsarm und unter Aufrechterhaltung einer gewünschten Positionierung zwischen einer bildgebenden Vorrichtung (zum Beispiel einem Computertomografen) und einer Vorrichtung zur Strahlentherapie bewegt werden kann. Hierzu können die Vorrichtungen allgemein auf einer gemeinsamen Seite oder gegenüberliegenden Seiten entlang der Linearachse angeordnet sein und/oder über Eck (eine Vorrichtung an einem axialen Ende der Linearachse und eine seitlich entlang der Linearachse). Auch ist eine gegenüberliegende Anordnung derartiger Vorrichtung an oder nahe entsprechenden Endbereichen der Linearachseinheit denkbar.

Die vorliegende Offenbarung soll weiter anhand von Figuren erläutert werden. Diese Figuren zeigen schematisch:
- Figuren 1 bis 3: perspektivische Darstellungen eines Roboters zur Patientenpositionierung gemäß eines Ausführungsbeispiels;
- Figur 3a: eine Explosionsdarstellung des Roboterarms des Roboters gemäß den Figuren 1 bis 3
- Figur 3b: eine Detailansicht einer Handgelenkseinheit des Roboters der Figuren 1 bis 3a;
- Figur 4: eine perspektivische Darstellung einer Roboteranordnung, umfassend einen Roboter gemäß des Ausführungsbeispiels der Figuren 1 bis 3 und eine Linearachseinheit;
- Figur 5a bis c: Draufsichten auf verschiedene Betriebszustände einer Roboterarbeitszelle umfassend eine Roboteranordnung gemäß Figur 4;
- Figur 6a bis c: perspektivische Darstellungen einer Roboterarbeitszelle analog zu den Figuren 5a bis c;
- Figur 7a bis b: perspektivische Darstellungen der Roboterarbeitszelle gemäß den vorangehenden Figuren, wobei der Roboter eine besonders bodennahe Stellung einnimmt; und
- Figur 8a bis b: Draufsichten auf eine Roboterarbeitszelle analog zu den vorangehenden Figuren, umfassend eine Mehrzahl von medizinischen Vorrichtungen.

Im Folgenden werden, ohne hierauf beschränkt zu sein, spezifische Details dargelegt, um ein vollständiges Verständnis der vorliegenden Offenbarung zu liefern. Es ist einem Fachmann jedoch klar, dass die vorliegende Offenbarung in anderen Ausführungsbeispielen verwendet werden kann, die von den nachfolgend dargelegten Details abweichen können.

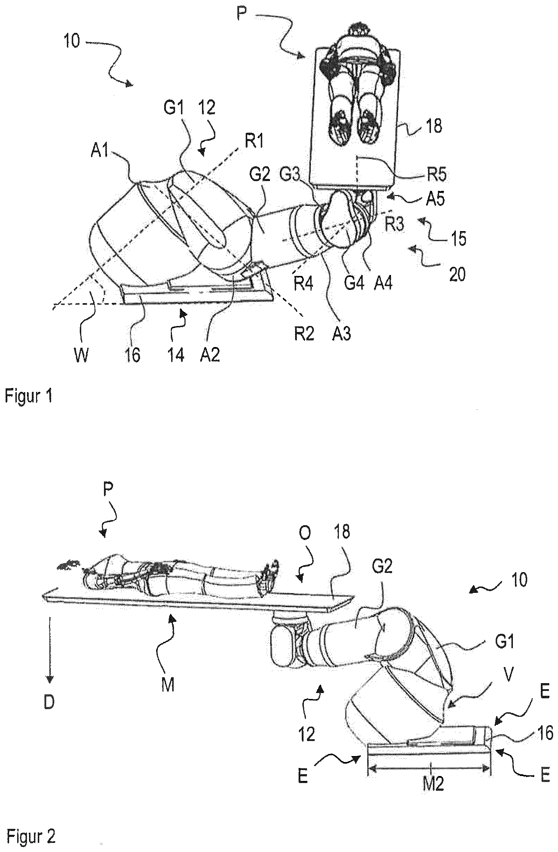

In Figur 1 ist ein Roboter 10 gemäß einem ersten Ausführungsbeispiel gezeigt. Der Roboter umfasst einen Roboterarm 12, der einen ersten Endbereich 14 umfasst, mit dem der Roboter 10 an einem nicht gesondert dargestellten Umgebungsbereich anordenbar und mit diesem mechanisch koppelbar ist. Dabei umfasst der erste Endbereich 14 ein plattenförmiges Bauteil 16, das in der nachstehend geschilderten Weise eine Anschlussebene sowie einen Kopplungsbereich zu einer horizontalen Raumebene bildet.

Ausgehend von dem plattenförmigen Bauteil 16 erstreckt sich der Roboterarm 12 im Sinne einer offenen kinematische Kette in den Raum hinein, wobei diese Kette einen zweiten Endbereich 15 mit einem Endeffektor in Form einer Patientenliege 18 aufweist. Diese ist in bekannter Weise mit dem zweiten Endbereich des Roboterarms 12 gekoppelt.

Im Folgenden wird die kinematische Struktur des Roboterarms 12 erläutert. Ausgehend von dem plattenförmigen Bauteil 16 umfasst der Roboterarm 12 zunächst eine erste Rotations-Achseinheit A1, die eine erste Rotationsachse R1 des Roboterarms 12 definiert. Die erste Rotations-Achseinheit A1, wie auch die nachfolgend erläuterten weiteren Rotations-Achseinheiten A2-A5, umfasst dabei ein per Motor, beispielsweise per Servomotor, angetriebenes Rotationsgelenk nach allgemein bekannter Bauart. Ausgehend von der ersten Achseinheit A1 erstreckt sich ein erstes Roboterglied G1, das zum Beispiel aus Metall und somit allgemein starr ausgebildet ist.

Das erste Roboterglied G1 erstreckt sich zu einer zweiten Rotations-Achseinheit A2, die eine zweite Rotationsachse R2 definiert. Man erkennt ferner, dass die zweite Rotationsachse R2 und die erste Rotationsachse R1 zwar durch das Roboterglied G1 voneinander beabstandet sind, sich jedoch senkrecht relativ zueinander erstrecken. Mit anderen Worten schließen diese Rotationsachsen R1, R2 zumindest in einem aufeinander projizierten Zustand einen rechten Winkel miteinander ein. Im gleichen Sinne können diese Achsen durch eine (nicht dargestellte) virtuelle Gerade verbunden werden, die senkrecht auf beiden Rotationsachsen R1, R2 steht.

Ausgehend von der zweiten Rotations-Achseinheit A2 erstreckt sich ein zweites Roboterglied G2 zu einer dritten Rotations-Achseinheit A3, die wiederum eine dritte Rotationsachse R3 definiert. Diese erstreckt sich orthogonal zu der zweiten Rotationsachse R2. Die Verbindung der zweiten und dritten Rotations-Achseinheiten A2, A3 erfolgt dabei über ein Turretsegment.

Die dritte Rotations-Achseinheit A3 ist Bestandteil einer Handgelenkseinheit 20 des Roboterarms 12, die in bekannter Weise zwei weitere (vierte und fünfte) Rotations-Achseinheiten A4, A5 mit dazugehörigen Rotationsachsen R4, R5 umfasst, die über dritte und vierte Roboterglieder G3 und G4 miteinander verbunden sind (in Figur 1 teilweise verdeckt). Die Rotationsachsen R3-R5 der Handgelenkseinheit 20 stehen ferner in bekannter Weise senkrecht auf der jeweils vorangehend und sind im Normalbetrieb (z. B. Anheben und Stützen der Patientenliege 18 von unten) derart angeordnet, dass sie im Wesentlich senkrecht zu sämtlichen weiteren Achsen der Handgelenkseinheit 20 ausgerichtet sind.

Als eine Besonderheit der vorliegenden Kinematik erkennt man in Figur 2, dass sich die erste Rotationsachse R1 schräg bezogen auf das plattenförmigen Bauteil 16 sowie die hiervon definierte Anschlussebene zur Umgebung erstreckt. Genauer gesagt verläuft die Rotationsachse R1 beispielhaft in einem Winkel W von 45° zu dem plattenförmigen Bauteil 16.

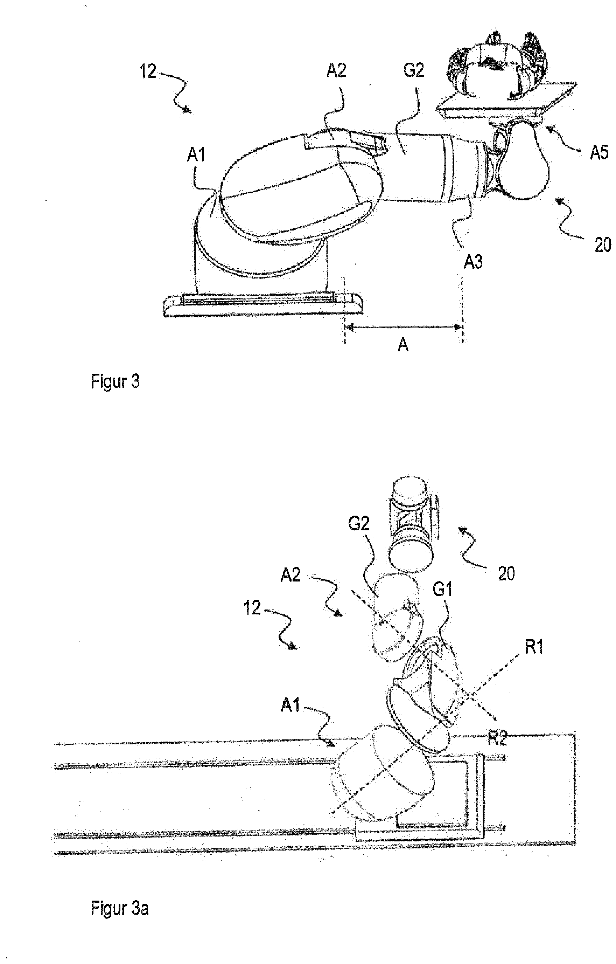

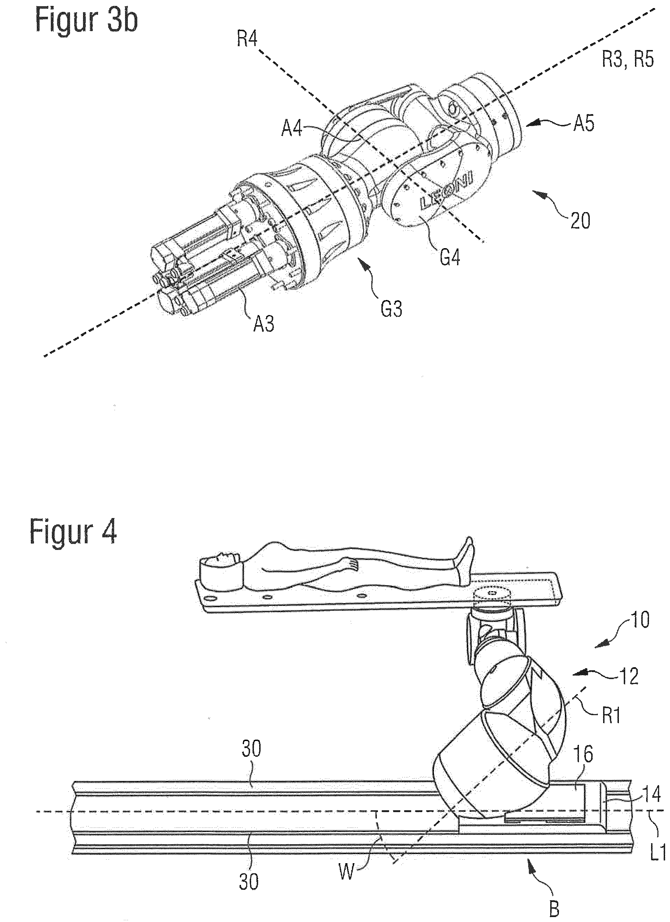

In den Figuren 2 und 3 ist der Roboter 10 aus Figur 1 mittels weiterer perspektivische Ansichten gezeigt, wobei der Roboterarm 12 jeweils unterschiedliche Betriebsstellungen einnimmt.

Insbesondere in Figur 2 erkennt man, dass der Roboterarm 12 mit seinem zweiten Anschlussbereich 15 nahe eines Randbereiches und somit exzentrisch von einer geometrischen Mitte M mit der Patientenliege 18 gekoppelt ist. Dabei hebt der Roboter 10 die Patientenliege 18 von unten an beziehungsweise stützt diese von unterhalb, so dass deren Oberseite O vollständig als Liegefläche für einen Patienten P genutzt werden kann. Entsprechend erfolgt eine Lasteinleitung in den Roboterarm 12 ebenfalls maßgeblich in exzentrischer Weise, wie durch die Druckbelastung D in Figur 2 angedeutet. Andererseits ergibt sich somit um den Patienten P ein großer Freiraum, der ein kollisionsfreies Anordnen der Patientenliege 18 nahe oder innerhalb von medizinischen Vorrichtungen ermöglicht.

In Figur 3 erkennt man ferner, dass das zweite Roboterglied G2 einen Abstand A zwischen den zweiten und dritten Rotations-Achseinheiten A2, A3 festlegt, der im vorliegenden Beispiel 1 m entspricht. Folglich ist auch die Handgelenkseinheit 20 des Roboterarms 12 in einem entsprechenden Abstand A zu den vorangehenden Rotations-Achseinheiten A1, A2 angeordnet.

Dies ermöglicht, dass die Handgelenkseinheit 20 beabstandet von den als Basis-Achsen dienenden Rotations-Achseinheiten A1, A2 angeordnet ist, die zudem vergleichsweise groß bauen, wodurch eine Störkontur nahe der Handgelenkseinheit 20 reduziert wird. Auch dies trägt zum Vermeiden von Kollisionen mit der Umgebung bei.

Zurückkommend auf Figur 2 wird ersichtlich, dass ein Verbindungsbereich V der ersten Rotations-Achseinheiten A1 mit dem ersten Roboterglied G1 oberhalb von beziehungsweise im Bereich der Anschlussebene angeordnet ist. Genauer gesagt befindet sich der Verbindungsbereich V etwa über einer geometrischen Mitte M2 des plattenförmigen Bauteils 16. Letzteres ist im Wesentlichen rechteckförmig ausgebildet und umfasst in jedem seiner Eckbereiche E nachfolgend erläuterte Kopplungselemente zur mechanischen Verbindung mit einer Linearachseinheit L (nicht sämtliche Eckbereiche E sind dabei in Figur 2 zu erkennen). Der Verbindungsbereich V liegt somit im Wesentlichen über einem Diagonalenschnittpunkt der Eckbereiche E beziehungsweise zwischen sämtlichen und insbesondere jeweils zwei einander gegenüberliegenden Kopplungselementen. Dies mindert die wirksamen Hebelarme und ermöglicht eine zuverlässige Abstützung und Krafteinleitung in das plattenförmigen Bauteil 16 sowie dem damit gekoppelten Umgebungsbereich.

In Figur 3a ist der Roboterarm 12 des Roboters 10 in einer Explosionsdarstellung gezeigt. Man erkennt hierbei noch einmal die Lage der einzelnen Achseinheiten A1-A5 sowie die Erstreckung der Roboterglieder G1-G4. Insbesondere wird ersichtlich, dass das die erste und zweite Achseinheit verbindende Roboterglied G1 in Form eines Turretsegments ausgebildet ist und die Rotationsachsen R1 und R2 relativ zueinander um 90° verdreht sind.

In Figur 3b ist schließlich eine Detaildarstellung der Handgelenkseinheit 20 gezeigt, die in bekannter Weise drei Achseinheiten A3-A5 umfasst. In der gezeigten Stellung fallen die dritte und fünfte Rotationsachse R3-R5 zusammen und stehen jeweils senkrecht auf der vierte Rotationsachse R4. Wie in Figur 1 gezeigt, sind im Normalbetrieb (Anheben und Stützen der Patientenliege 18 von unten) die Rotationsachsen R3-R5 jedoch derart angeordnet, dass sämtliche Rotationsachsen R3-R5 im Wesentlichen senkrecht relativ zueinander verlaufen.

Figur 4 zeigt eine Roboteranordnung, umfassend einen Roboter 10, wie vorstehend anhand der Figuren 1-3 erläutert, sowie eine Linearachseinheit L. Die Linearachseinheit L umfasst zwei zueinander parallele Führungsschienen 30 und ist insgesamt auf einer horizontalen ebenen Bodenfläche B im Raum angeordnet. Entsprechend bildet die Linearachseinheit L einen Umgebungsbereich in Form einer horizontalen Raumebene, an der Roboter 10 angeordnet ist. Ferner definiert sie eine lineare (bzw. translatorische) Bewegungsachse L1, die sich ebenfalls parallel zu der ebenen Bodenfläche B erstreckt und entlang derer der Roboter 10 im Raum verschoben werden kann. Hierzu ist der Roboterarm 12 über das plattenförmigen Bauteil 16 und, wie vorstehend erläutert, den daran angeordneten Kopplungselementen mit Antriebseinheiten der Linearachseinheit L verbunden, die eine Bewegung entlang der Linearachse L1 ermöglichen . Die Antriebseinheiten können beispielsweise als Linearmotoren ausgebildet sein und die Kopplungselemente können Verschraubungsbolzen, Klebestellen oder sonstige Befestigungselemente umfassen, um den Roboter 10 hiermit zu koppeln.

In Figur 4 ist ferner erneut die Neigung der ersten Rotationsachse R1 des Roboterarms 12 in Form des Winkels W zu dem plattenförmigen Bauteil 16 beziehungsweise zu der Anschlussebene des ersten Endbereichs 14 gezeigt. Man erkennt, dass die Rotationsachse R1 den gleichen Winkel W mit der Linearachseinheit L sowie der Linearachse L1 einschließt und somit auch mit der dazu parallelen ebenen Bodenfläche B.

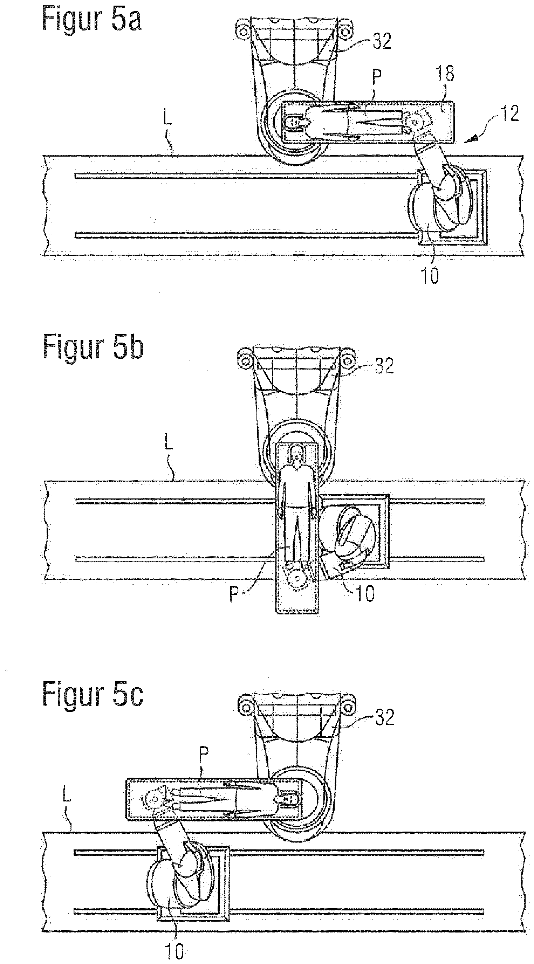

In den Figuren 5a-c sind Draufsichten auf verschiedene Betriebszustände einer Roboterarbeitszelle gezeigt, die eine Roboteranordnung gemäß Figur 4 sowie eine medizinische Strahlentherapievorrichtung 32 umfasst. Konkret erkennt man erneut den Roboter 10 mit der daran angebrachten Patientenliege 18 sowie die Linearachseinheit L zum Verschieben des Roboters 10 im Raum. Die Figuren 5a-c zeigen dabei beispielhaft verschiedene Stellungen der einzelnen Achsen R1-R5 und der Roboterglieder G1-G4 des Roboterarms 12, mittels derer ein Patient P (und im gezeigten Fall dessen Kopfbereich) in einem Bestrahlungs-Arbeitsbereich der medizinischen Vorrichtung 32 positioniert werden kann. Dabei kann auch eine kontinuierliche Bewegung zwischen den in den Figuren 5a-c gezeigten Stellungen durch koordinierte Ansteuerung der einzelnen Roboter-Achseinheiten A1-A5 und der Linearachseinheit L erfolgen. Insbesondere aus Figur 5b verdeutlicht sich, dass der Roboter 10 aufgrund seiner kompakten Gestalt eine geringe Störkontur bei ausreichender Steifigkeit aufweist und somit kollisionsfrei nahe und entlang der medizinischen Vorrichtung 32 bewegbar ist.

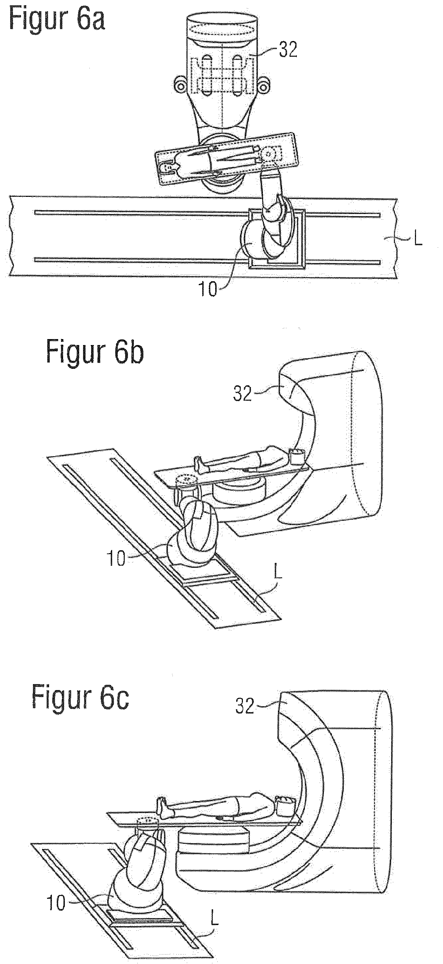

Die Figuren 6a-6c zeigen analog zu den Figuren 5a-c weitere perspektivische Darstellungen der Roboterarbeitszelle in verschiedenen Betriebszuständen, bei der ein Patient P mittels des Roboters 10 in gewünschter Weise relativ zu der Strahlentherapievorrichtung 32 positioniert und bewegt wird.

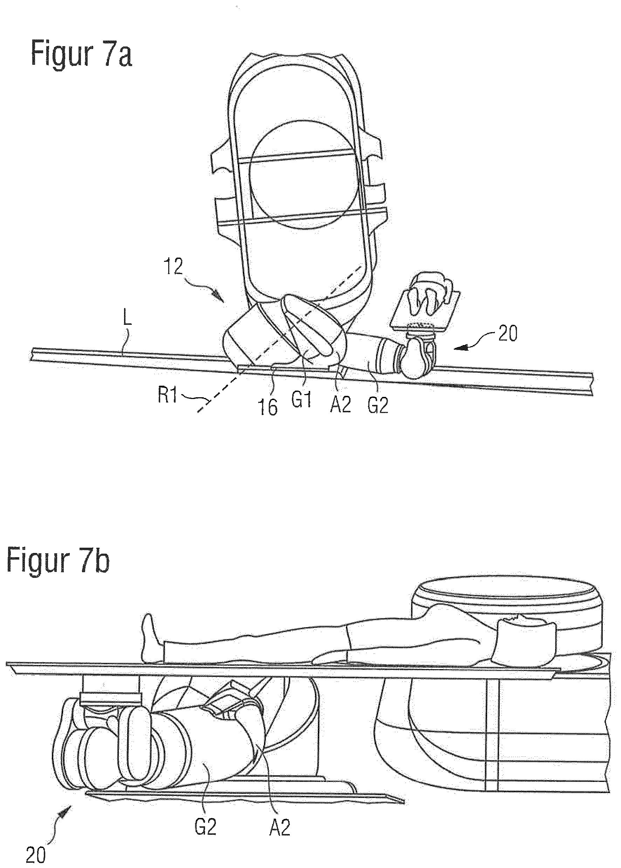

In den Figuren 7a-b sind perspektivische Darstellungen der Roboterarbeitszelle gemäß den vorangehenden Figuren gezeigt, wobei der Roboter 10 eine besonders bodennahe Stellung einnimmt. Genauer gesagt erkennt man, dass das erste Roboterglied G1 mittels der ersten Rotationsachse R1 in Richtung der Linearachseinheit L rotiert wurde, sodass die zweite Rotations-Achseinheiten A2 unmittelbar oberhalb des plattenförmigen Bauteils 16 angeordnet ist. Hiervon ausgehend erstreckt sich das zweite Roboterglied G2 im Wesentlichen geradlinig, sodass die Handgelenkseinheit 20 des Roboterarms 12 in geringer Höhe (beziehungsweise in einem geringen vertikalen Abstand) zu einem Bodenbereich angeordnet ist.

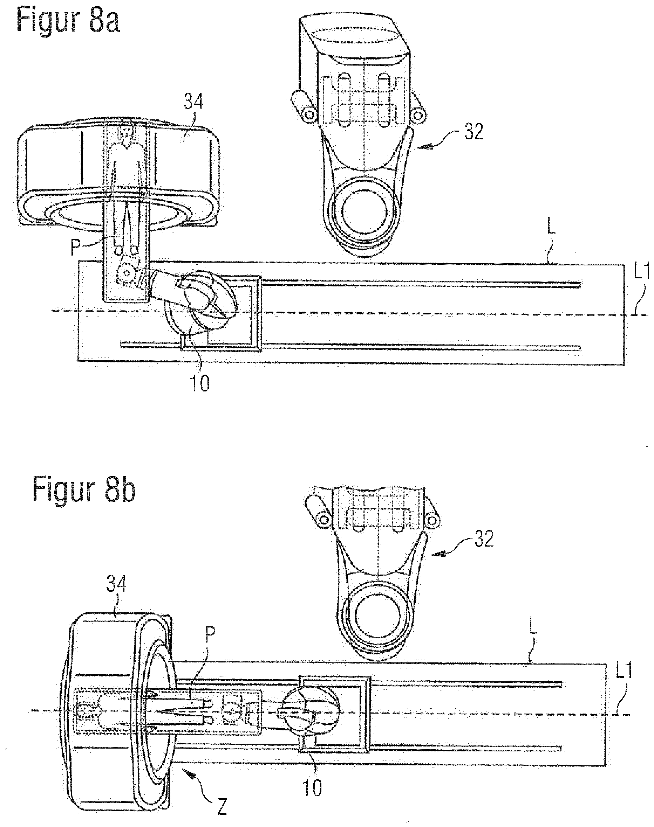

Die Figuren 8a-b zeigen schließlich Draufsichten auf eine Roboterarbeitszelle analog zu den vorangehenden Figuren, umfassend eine Mehrzahl von medizinischen Vorrichtungen 32, 34. Konkret umfassen die Roboterarbeitszellen jeweils eine Strahlentherapievorrichtung 32 sowie einen bildgebenden Computertomografen 34. Im in Figur 8a gezeigten Fall sind beide medizinischen Vorrichtungen 32, 34 auf einer gemeinsamen Seite entlang der Linearachse L1 angeordnet. Dabei ist die Linearachseinheit L derart angeordnet, dass der Roboter 10 einen Patienten P wahlweise im Arbeitsbereich einer der Vorrichtungen 32, 34 positionieren kann.

Im Fall von Figur 8b ist der Computertomograf 34 an einem axialen Ende Z der Linearachseinheit L angeordnet, sodass der Patient P nach einer entsprechenden Ausrichtung über den Roboter 10 (insbesondere parallel zur Linearachse L1) mittels der Linearachseinheit L in den Computertomografen 34 hineingeschoben werden kann. Gleichzeitig ist aber nach wie vor ein Anordnen des Patienten P im Arbeitsbereich der Strahlentherapievorrichtung 32 möglich. Hierzu kann der Patient P mittels der Roboteranordnung analog zu den in den Figurengruppe 5 und 6 gezeigten Betriebsstellungen ausgerichtet werden.

## Patentansprüche

1. Roboter (10), beispielsweise zur Patientenpositionierung, umfassend einen Roboterarm (12) mit einer Mehrzahl von Robotergliedern (G1-G4), die über Achseinheiten (A1-A5) miteinander verbunden sind,
wobei die Achseinheiten (A1-A5) jeweils wenigstens eine Bewegungsachse des Roboterarms (12) definieren,
wobei der Roboterarm (12) einen ersten Endbereich (14) umfasst, der ein Anordnen an einem Umgebungsbereich des Roboters (10) ermöglicht, und einen zweiten Endbereich (15), an dem ein Endeffektor anordenbar ist,
wobei eine dem ersten Endbereich (14) nachgeordnete erste Achseinheit eine erste Rotationsachse (R1) des Roboterarms (12) definiert, und
wobei der Roboterarm (12) mittels des ersten Endbereichs (14) derart an dem Umgebungsbereich anordenbar ist, dass die erste Rotationsachse (R1) in einem Winkel (W) schräg zu dem Umgebungsbereich verläuft,
wobei der Umgebungsbereich eine im Wesentlichen horizontale Raumebene umfasst und der Roboter (10) dazu ausgebildet ist, den Roboterarm (12) mittels des ersten Endbereichs (14) an der horizontalen Raumebene unter Bereitstellen des Winkels (W) der ersten Rotationsachse (R1) zu befestigen.

2. Roboter (10) nach Anspruch 1,
wobei die erste Rotationsachse (A1) in einem Winkel zwischen ca. 10° und ca. 80°, zum Beispiel in einem Winkel von ca. 20° bis ca. 70°, beispielsweise ca. 45°, zu dem Umgebungsbereich verläuft.

3. Roboter (10) nach einem der vorangehenden Ansprüche,
wobei der erste Endbereich (14) eine Anschlussebene umfasst, die im Wesentlichen einen selben Winkel (W) mit der ersten Rotationsachse (R1) einschließt wie der Umgebungsbereich.

4. Roboter (10) nach einem der vorangehenden Ansprüche,
wobei der Umgebungsbereich eine Bodenebene umfasst.

5. Roboter (10) nach einem der vorangehenden Ansprüche,
wobei die Anschlussebene über wenigstens zwei Kopplungselemente mit dem Umgebungsbereich gekoppelt ist und ein Verbindungsbereich der ersten Achseinheit (A1) mit einem ersten Roboterglied (G1) im Wesentlichen zwischen den Kopplungselementen angeordnet ist.

6. Roboter (10) nach einem der vorangehenden Ansprüche,
wobei der Roboterarm (12) ferner eine der ersten Achseinheit (A1) nachgeordnete zweite Achseinheit (A2) umfasst, die eine zweite Rotationsachse (R2) des Roboterarms (12) definiert.

7. Roboter (10) nach einem der vorangehenden Ansprüche,
wobei der Roboterarm (12) dazu ausgebildet ist, die zweite Achseinheit (A2) nahe des Umgebungsbereiches zu positionieren.

8. Roboter (10) nach einem der vorangehenden Ansprüche,
wobei der Roboterarm (12) eine der ersten und zweiten Achseinheit (A1, A2) nachgeordnete dritte Achseinheit (A3) umfasst, die eine dritte Rotationsachse (R3) des Roboterarms (12) definiert, und, optional, ferner der dritten Achseinheit (A3) nachgeordnete vierte und fünfte Achseinheiten (A4, A5), die entsprechende vierte und fünfte Rotationsachsen (R4, R5) des Roboterarms (12) definieren.

9. Roboter (10) nach Anspruch 8,
wobei die dritte Rotationsachse (R3) im Wesentlichen senkrecht zu der zweiten Rotationsachse (R2) verläuft.

10. Roboter (10) nach Anspruch 8 oder 9,
wobei die zweite und dritte Achseinheit (A2, A3) zwischen ca. 30 cm und ca. 2 m voneinander beabstandet sind und beispielsweise zwischen ca. 60 cm und ca. 1,50 m.

11. Roboteranordnung,
umfassend einen Roboter (10) nach einem der vorangehenden Ansprüche,
wobei der Umgebungsbereich eine Linearachseinheit (L) umfasst, an der der Roboterarm (12) über dessen ersten Endbereich (14) anordenbar ist.

12. Roboteranordnung nach Anspruch 11,
wobei die Linearachseinheit (L) eine lineare Bewegungsachse definiert, die einen selben Winkel (W) mit der ersten Rotationsachse (R1) des Roboters (10) einschließt wie der Umgebungsbereich.

13. Roboteranordnung nach Anspruch 11 oder 12,
wobei die Linearachseinheit (L) an einem Bodenbereich anordenbar ist.

14. Roboterarbeitszelle,
umfassend einen Bodenbereich und eine Roboteranordnung nach einem der Ansprüche 11 bis 13, wobei die Linearachseinheit (L) an dem Bodenbereich angeordnet ist.

15. Roboterarbeitszelle nach Anspruch 14,
ferner umfassend eine bildgebende medizinische Untersuchungsvorrichtung (34) und/oder eine Vorrichtung zur medizinischen Strahlentherapie (32), wobei die Roboteranordnung dazu ausgebildet ist, einen Patienten in einem Arbeitsbereich der medizinischen Vorrichtung (32, 34) zu positionieren.

## Claims

1. Robot (10), for example for patient positioning, comprising a robot arm (12) with a plurality of robot members (G1-G4) connected to each other via axis units (A1-A5),
wherein the axis units (A1-A5) each define at least one axis of movement of the robot arm (12),
wherein the robot arm (12) comprises a first end portion (14) that allows placement on a surrounding area of the robot (10) and a second end portion (15) on which an end effector can be placed,
wherein a first axis unit located downstream of the first end portion (14) defines a first axis of rotation (R1) of the robot arm (12), and
wherein the robot arm (12) can be arranged on the surrounding area by means of the first end portion (14) in such a way that the first axis of rotation (R1) runs at an angle (W) transverse to the surrounding area,
wherein the surrounding area comprises a substantially horizontal spatial plane and the robot (10) is adapted to attach the robot arm (12) by means of the first end portion (14) to the horizontal spatial plane while providing the angle (W) of the first axis of rotation (R1).

2. Robot (10) according to claim 1,
wherein the first axis of rotation (A1) extends at an angle between about 10° and about 80°, for example at an angle of about 20° to about 70°, for example about 45°, with respect to the surrounding area.

3. Robot (10) according to any of the preceding claims,
wherein the first end portion (14) comprises a connection plane which encloses substantially a same angle (W) with the first axis of rotation (R1) as the surrounding area.

4. Robot (10) according to any of the preceding claims,
wherein the surrounding area comprises a ground level.

5. Robot (10) according to any of the preceding claims,
wherein the connection plane is coupled to the surrounding area via at least two coupling elements, and a connection area of the first axis unit (A1) with a first robot member (G1) is arranged substantially between the coupling elements.

6. Robot (10) according to any of the preceding claims,
wherein the robot arm (12) further comprises a second axis unit (A2) downstream of the first axis unit (A1) and defining a second axis of rotation (R2) of the robot arm (12).

7. Robot (10) according to any of the preceding claims,
wherein the robot arm (12) is adapted to position the second axis unit (A2) close to the surrounding area.

8. Robot (10) according to any one of the preceding claims,
wherein the robot arm (12) comprises a third axis unit (A3) downstream of the first and second axis units (A1, A2) defining a third axis of rotation (R3) of the robot arm (12), and, optionally, further fourth and fifth axis units (A4, A5) downstream of the third axis unit (A3) defining respective fourth and fifth axes of rotation (R4, R5) of the robot arm (12).

9. Robot (10) according to claim 8,
wherein the third axis of rotation (R3) is substantially perpendicular to the second axis of rotation (R2).

10. Robot (10) according to claim 8 or 9,
wherein the second and third axis units (A2, A3) are spaced between about 30 cm and about 2 m from one another, and for example between about 60 cm and about 1.50 m.

11. Robot arrangement,
comprising a robot (10) according to any one of the preceding claims,
wherein the surrounding area comprises a linear axis unit (L) on which the robot arm (12) can be arranged via its first end portion (14).

12. A robot assembly according to claim 11,
wherein the linear axis unit (L) defines a linear axis of motion which encloses a same angle (W) with the first axis of rotation (R1) of the robot (10) as the surrounding area.

13. A robot assembly according to claim 11 or 12,
wherein the linear axis unit (L) can be arranged on a floor area.

14. Robot work cell,
comprising a floor area and a robot assembly according to any one of claims 11 to 13, wherein the linear axis unit (L) is arranged at the floor area.

15. The robotic work cell of claim 14,
further comprising a medical imaging examination device (34) and/or a medical radiation therapy device (32), wherein the robot assembly is adapted to position a patient in a working area of the medical device (32, 34).

## Revendications

1. Robot (10), par exemple pour le positionnement de patients, comprenant un bras de robot (12) avec une pluralité de membres de robot (G1-G4) qui sont reliés entre eux par des unités d'axe (A1-A5),
dans lequel les unités d'axe (A1-A5) définissent chacune au moins un axe de mouvement du bras de robot (12),
dans lequel le bras de robot (12) comprend une première zone d'extrémité (14) qui permet de le disposer sur une zone environnante du robot (10) et une deuxième zone d'extrémité (15) sur laquelle un effecteur final peut être disposé,
dans lequel une première unité d'axe disposée en aval de la première zone d'extrémité (14) définit un premier axe de rotation (R1) du bras de robot (12), et
dans lequel le bras de robot (12) peut être disposé sur la zone environnante au moyen de la première zone d'extrémité (14) de telle sorte que le premier axe de rotation (R1) s'étende selon un angle (W) obliquement par rapport à la zone environnante,
dans lequel la zone environnante comprend un plan spatial sensiblement horizontal et le robot (10) est conçu pour fixer le bras de robot (12) au plan spatial horizontal au moyen de la première zone d'extrémité (14) en fournissant l'angle (W) du premier axe de rotation (R1).

2. Robot (10) selon la revendication 1,
dans lequel le premier axe de rotation (A1) s'étend selon un angle compris entre environ 10° et environ 80°, par exemple selon un angle d'environ 20° à environ 70°, par exemple d'environ 45°, par rapport à la zone environnante.

3. Robot (10) selon l'une des revendications précédentes,
dans lequel la première zone d'extrémité (14) comprend un plan de raccordement qui fait sensiblement le même angle (W) avec le premier axe de rotation (R1) que la partie environnante.

4. Robot (10) selon l'une des revendications précédentes,
dans lequel la zone environnante comprend un plan de sol.

5. Robot (10) selon l'une des revendications précédentes,
dans lequel le plan de raccordement est couplé à la zone environnante par l'intermédiaire d'au moins deux éléments de couplage et une zone de liaison de la première unité d'axe (A1) avec un premier membre de robot (G1) est disposée sensiblement entre les éléments de couplage.

6. Robot (10) selon l'une des revendications précédentes,
dans lequel le bras de robot (12) comprend en outre une deuxième unité d'axe (A2) disposée en aval de la première unité d'axe (A1), qui définit un deuxième axe de rotation (R2) du bras de robot (12).

7. Robot (10) selon l'une des revendications précédentes,
dans lequel le bras de robot (12) est conçu pour positionner la deuxième unité d'axe (A2) à proximité de la zone environnante.

8. Robot (10) selon l'une des revendications précédentes,
dans lequel le bras de robot (12) comprend une troisième unité d'axe (A3) disposée en aval des première et deuxième unités d'axe (A1, A2), qui définit un troisième axe de rotation (R3) du bras de robot (12), et, éventuellement, en outre des quatrième et cinquième unités d'axe (A4, A5) disposées en aval de la troisième unité d'axe (A3), qui définissent des quatrième et cinquième axes de rotation (R4, R5) correspondants du bras de robot (12).

9. Robot (10) selon la revendication 8,
dans lequel le troisième axe de rotation (R3) est sensiblement perpendiculaire au deuxième axe de rotation (R2).

10. Robot (10) selon la revendication 8 ou 9,
dans lequel les deuxième et troisième unités d'axe (A2, A3) sont espacées l'une de l'autre entre environ 30 cm et environ 2 m, et, par exemple, entre environ 60 cm et environ 1,50 m.

11. Ensemble robotique,
comprenant un robot (10) selon l'une des revendications précédentes,
dans lequel la zone environnante comprend une unité d'axe linéaire (L) sur laquelle le bras de robot (12) peut être disposé par l'intermédiaire de sa première zone d'extrémité (14).

12. Ensemble robotique selon la revendication 11,
dans lequel l'unité d'axe linéaire (L) définit un axe de mouvement linéaire qui fait le même angle (W) avec le premier axe de rotation (R1) du robot (10) que la zone environnante.

13. Ensemble robotique selon la revendication 11 ou 12,
dans lequel l'unité d'axe linéaire (L) peut être disposée sur une zone de sol.

14. Cellule de travail robotique,
comprenant une zone de sol et un ensemble robotique selon l'une des revendications 11 à 13, dans laquelle l'unité d'axe linéaire (L) peut être disposée sur la zone de sol.

15. Cellule de travail robotique selon la revendication 14,
comprenant en outre un dispositif médical d'examen par imagerie (34) et/ou un dispositif médical de radiothérapie (32), dans laquelle l'ensemble robotique est conçu pour positionner un patient dans une zone de travail du dispositif médical (32, 34).
